# EUROPEAN PATENT APPLICATION

(11) **EP 2 982 746 A1**
(43) Date of publication of application: **10.02.2016**
(21) Application number: 14002764.0
(22) Date of filing: 07.08.2014
(51) Int. Cl.: C12N 5/0783

(54) **Regulatory T cells with therapeutic potential**

(71) Applicant: TXCell, 06560 Valbonne (FR)
(72) Inventor: Foussat, Arnaud, 06560 Valbonne (FR); Belmonte, Nathalie, 06200 Nice (FR); Gertner - Dardenne, Julie, 06130 Grasse (FR)

(57) **Abstract**

The present invention relates to a Treg cell population with therapeutic potential, wherein said population expresses at least 3 cytotoxic molecules selected from the group comprising granulysin, granzyme A, granzyme B, granzyme M, granzyme K, granzyme H and perforin. The present invention further relates to a method for identifying or selecting a Treg cell population with therapeutic potential, and to the use of said Treg cell populations for treating an unwanted immune response associated with transplantation or an allergic disease or an inflammatory condition or an autoimmune condition.

## Description

### FIELD OF INVENTION

The present invention relates to Treg cell populations that may be used in cell therapy, wherein the administration of these Treg cell populations to a patient is predicted to induce a therapeutic benefit of said patient. In particular, the Treg cell populations of the invention are characterized by expression of multiple cytotoxic molecules and specific expression level of these cytotoxic molecules.

### BACKGROUND OF INVENTION

The immune system is a complicated network of many different players, which interact with each other and cooperate to protect against diseases and fight established diseases. Among these players are regulatory T cells which act to suppress immune activation and thereby maintain immune homeostasis and tolerance to self-antigens.

Regulatory T cells are described in the art to comprise distinct cell populations such as thymus-derived regulatory Treg cells (tTreg), and peripherally-derived Treg cells (pTreg).

Although the therapeutic potential of regulatory T cells was envisioned decades ago, clinical induction of their potent immune regulatory activity in patients by in vivo administration of therapeutics chemical or biologic agents has proven challenging. Adoptive regulatory T cells therapy is an attractive alternative to harness the immune suppressive activity of regulatory T cells. In this approach, regulatory T cells are isolated from a patient or a healthy donor, enriched, sometimes further expanded ex vivo, and reinfused either to the same patient or to allogeneic recipients.

During cell therapy, different Treg cell populations may be obtained by in vitro manipulation of cells harvested from a subject. Examples of in vitro manipulations include, but are not limited to, isolation, expansion, induction of proliferation, activation of naive Treg cells via exposure to an antigen, differentiation of naïve T cells into regulatory T cells and the like. However, the therapeutic potential of all Treg populations do not seem to be equivalent: therefore, during therapy, some Treg populations effectively induce a therapeutic response of the patient, whereas other don't. There is thus a need for methods for selecting Treg populations based on their therapeutic potential, and for predicting the therapeutic response of the patient to the Treg cell therapy.

The Inventors compared Treg populations resulting or not in a therapeutic response of a patient to a Treg cell therapy, and surprisingly identified the expression of cytotoxic molecules as a biomarker of the therapeutic potential of said Treg populations.

The present invention thus relates to specific subpopulations of Treg cells with therapeutic potential, and to a method for identifying said Treg cell populations, based on the expression of cytotoxic molecules.

### SUMMARY

The present invention thus relates to a Treg cell population expressing at least 3 cytotoxic molecules selected from the group comprising granulysin, granzyme A, granzyme B, granzyme M, granzyme K, granzyme H and perforin.

In one embodiment, said at least three cytotoxic molecules comprise granzyme B, preferably granzyme B and granzyme H.

In one embodiment, the Treg cell population of the invention is characterized in that the score resulting from the sum of:
- the percentage of cells of the Treg cell population expressing a first cytotoxic molecule, and
- the percentage of cells of the Treg cell population expressing a second cytotoxic molecule,
- the percentage of cells of the Treg cell population expressing a third cytotoxic molecule,
- optionally the percentage of cells of the Treg cell population expressing a fourth cytotoxic molecule,
- optionally the percentage of cells of the Treg cell population expressing a fifth cytotoxic molecule,
- optionally the percentage of cells of the Treg cell population expressing a sixth cytotoxic molecule, and
- optionally the percentage of cells of the Treg cell population expressing a seventh cytotoxic molecule,
is superior to a reference score measured in Treg cell populations that do not induce a therapeutic response when used in a Treg cell therapy in a patient.

In another embodiment, the Treg cell population of the invention is characterized in that
- at least 30% of the cells of the Treg population express granzyme B,
- at least 80% of the cells of the Treg population express granzyme H, and
- at least 30% of the cells of the Treg population express at least one cytotoxic molecule selected from granulysin, granzyme A, granzyme K, granzyme M and perforin.

In one embodiment, the Treg cell population of the invention is a Tr1 cell population. In one embodiment, the Treg cell population of the invention is an antigen-specific Treg cell population.

The present invention also relates to a method for identifying Treg cells as hereinabove described, comprising determining the expression level of cytotoxic molecules, wherein said expression level of cytotoxic molecules comprises the expression level of at least 3 cytotoxic molecules selected from the group comprising granulysin, granzyme A, granzyme B, granzyme M, granzyme K, granzyme H and perforin, wherein the Treg cells expressing at least three cytotoxic molecules are Treg cells with therapeutic potential.

In one embodiment, said at least three cytotoxic molecules comprise granzyme B, preferably granzyme B and granzyme H.

In one embodiment, said method comprises:
a. determining in the Treg cell population to be administered to the subject:
   - the percentage of cells expressing a first cytotoxic molecule,
   - the percentage of cells expressing a second cytotoxic molecule,
   - the percentage of cells expressing a third cytotoxic molecule,
   - optionally the percentage of cells expressing a fourth cytotoxic molecule,
   - optionally the percentage of cells expressing a fifth cytotoxic molecule,
   - optionally the percentage of cells expressing a sixth cytotoxic molecule, and
   - optionally the percentage of cells expressing a seventh cytotoxic molecule,
b. summing the at least three percentages measured in step (a), thereby obtaining a score, and
c. comparing said score with a reference score.

In one embodiment, a Treg cell population comprising:
- more than 30% of cells expressing granzyme B
- more than 80% of cells expressing granzyme H, and
- more than 30% of cells expressing at least one of granulysin, granzyme A, granzyme M, granzyme K and perforin,
is a Treg cell population with therapeutic potential.

In one embodiment, the Treg cell population is a Tr1 cell population. In one embodiment, the Treg cell population is an antigen-specific Treg cell population.

Another object of the invention is a Treg cell population identified by the method of the invention.

The present invention also relates to a Treg cell population as hereinabove described, for use for treating an inflammatory condition, an autoimmune condition or an immune response associated with transplantation or with an allergic disease

Another object of the invention is a kit for implementing the method for identifying Treg cells with therapeutic potential as hereinabove described, wherein said kit comprises means for measuring the expression of at least three cytotoxic molecules, preferably wherein said means are antibodies specific for said at least three cytotoxic molecules.

### DEFINITIONS

In the present invention, the following terms have the following meanings:
- The term "regulatory T cells (or Treg cells)" refers to cells that suppress, inhibit or prevent excessive or unwanted inflammatory responses, such as, for example, autoimmunity or allergic reactions. Regulatory T cells include
   - thymus-derived Treg cells (tTreg, previously referred as "natural Treg cells"). As used herein, tTregs have the following phenotype at rest CD4⁺CD25⁺FoxP3⁺,
   - peripherally-derived Treg cells (pTreg, previously referred as "induced Treg cells"), including, for example, Tr1 cells, TGF-β secreting Th3 cells, regulatory NKT cells, regulatory γδ T cells, regulatory CD8⁺ T cells, double negative regulatory T cells,
   - in vitro-induced Treg cells and
   - engineered Treg cells.
- The term "Tr1 cells" as used herein refers to cells having the following phenotype at rest: CD4⁺CD25⁻ CD127⁻, and the following phenotype when activated: CD4⁺CD25⁺ CD127⁻. Tr1 cells, Type 1 T regulatory cells (Type 1 Treg) and IL-10 producing Treg are used herein with the same meaning. In one embodiment, Tr1 cells may be characterized, in part, by their unique cytokine profile: they produce IL-10, and IFN-gamma, but little or no IL-4 or IL-2. In one embodiment, Tr1 cells are also capable of producing IL-13 upon activation. The cytokine production is typically evaluated in cultures of cells after activation with polyclonal activators of T lymphocytes such as anti-CD3 + anti-CD28 antibodies or Interleukin-2, PMA + ionomycin. Alternatively, the cytokine production is evaluated in cultures of cells after activation with the specific T-cell antigen presented by antigen presenting cells. Regarding production of IL-10, Tr1 cells may produce at least about 50 pg/ml, typically more than about 100, 500 pg/mL or more than about 1, 2, 4, 6, 8, 10, 12, 14, 16, 18, or 20 thousand pg/ml or more. Regarding production of IFN-gamma, Tr1 cells may produce concentrations comprised between 0 pg/ml and at least 400 pg/ml, or of more than about 400 pg/mL, typically greater than about 600, 800, 1000, 1200, 1400, 1600, 1800, or 2000 pg/ml or more. Little or no IL-4 or IL-2 corresponds to less than about 1000 pg/ml, preferably less than about 750, 500, 250, 100, 75, or 50 pg/ml, or less. Regarding the production of IL-13, Tr1 cells may produce at least about 1 ng/ml, typically at least about 10, 25, 50, or 80 ng/ml.
- The term "Th3 cells" as used herein refers to cells having the following phenotype CD4⁺FoxP3⁺ and capable of secreting high levels TGF-β upon activation, low amounts of IL-4 and IL-10 and no IFN-γ or IL-2. These cells are TGF-β derived.
- The term "regulatory NKT cells" as used herein refers to cells having the following phenotype at rest CD161⁺CD56⁺CD16⁺ and expressing a Vα24/Vβ11 TCR.
- The term "regulatory CD8⁺ T cells" as used herein refers to cells having the following phenotype at rest CD8⁺CD122⁺ and capable of secreting high levels of IL-10 upon activation.
- The term "double negative regulatory T cells" as used herein refers to cells having the following phenotype at rest TCRαβ⁺CD4⁻CD8⁻.
- The term "in vitro inducible regulatory T cells" as used herein refers to naive T cells that are differentiated into regulatory T cells in vitro. One example of said in vitro inducible regulatory T cells is Th3 cells that are differentiated from naive T cells in the presence of TGF-β. Other examples are natural regulatory T cells or Tr1 cells obtained by in vitro differentiation.
- The term "γδ T cells" as used herein refers to T lymphocytes that express the [gamma] [delta] heterodimer of the TCR. Unlike the [alpha] [beta] T lymphocytes, they recognize non-peptide antigens via a mechanism independent of presentation by MHC molecules. Two populations of γδ T cells may be described: the yδ T lymphocytes with the V γ9V δ2 receptor, which represent the majority population in peripheral blood and the γδ T lymphocytes with the V δ1 receptor, which represent the majority population in the mucosa and have only a very limited presence in peripheral blood. V γ9V δ2 T lymphocytes are known to be involved in the immune response against intracellular pathogens and hematological diseases.
- The term "engineered Treg cells" as used herein refers to T lymphocytes genetically engineered, such as, for example T lymphocytes wherein the expression of transgenes has been induced, thereby obtaining regulatory T cells. This term also encompasses Treg cell populations that have been modified by introduction of transgenes or deletion or inhibition of expression or translation of endogenous genes.
- The term "cytotoxic molecules" refers to a protein involved in the cytotoxic pathway, preferably to a protein of the serine protease family or to the granzyme family or to granulyzin or to perforin. The granzyme family is a family of serine proteases that are released by cytoplasmic vesicles, which can be found for example within cytotoxic T cells and natural killer cells. In one embodiment, the protease from the granzyme family is selected from the group comprising granzyme A, granzyme B, granzyme M, granzyme K and granzyme H. Granulysin is a substance first described as released by cytotoxic T cells (CD8) when they are attached to infected body cells. Granulysin may create holes in a target cell membrane, but also induce apoptosis in target cells. Perforin is a cytolytic protein found in the secretory vesicles of Cytotoxic T lymphocytes (CTLs) and NK cells. Upon degranulation, perforin inserts itself into the target cell's plasma membrane, forming a pore.
- The term "treatment" as used herein refers to therapeutic treatment and prophylactic and preventive measures, wherein the object is to prevent or slow down (lessen, diminish) the targeted pathological disorder or condition. Those in need of treatment include those already with the disorder as well as those prone to have the disorder or those in whom the disorder is to be prevented. A subject or mammal is successfully "treated" for a disease if, after receiving a therapeutic amount of Treg cells according to the present invention, the patient shows observable and/or measurable reduction in or absence of one or more of the following: reduction in the number of pathogenic cells; reduction in the percent of total cells that are pathogenic; and/or relief to some extent, of one or more of the symptoms associated with the specific disease or condition; reduced morbidity and mortality, and improvement in quality of life issues. The above parameters for assessing successful treatment and improvement in the disease are readily measurable by routine procedures familiar to a physician. Treg cell treatment and Treg cell therapy are used herein with the same meaning.

- The term "therapeutically effective amount" refers to the number of Treg cells that is aimed at inducing a therapeutic response, without causing significant negative or adverse side effects to the target. A therapeutically effective amount may be administered prior to the onset of the disease to be treated, for a prophylactic or preventive action. Alternatively or additionally, the therapeutically effective amount may be administered after initiation of the disease to be treated, for a therapeutic action.
- The term "therapeutic response" refers to a therapeutic benefit induced by a Treg cell therapy in a subject. A therapeutic response may include the fact of (1) delaying or preventing the onset of the disease to be treated; (2) slowing down or stopping the progression, aggravation, or deterioration of one or more symptoms of the disease to be treated; (3) bringing about ameliorations of the symptoms of the disease to be treated; (4) reducing the severity or incidence of the disease to be treated; or (5) curing the disease to be treated.
- As used herein, the term "about" preceding a figure means more or less 10% of the value of said figure.
- The term "patient" refers to a mammal, preferably a human, who/which is awaiting the receipt of, or is receiving a Treg cell therapy. In the present invention, the terms subject and patient may be used with the same meaning.

### DETAILED DESCRIPTION

The present invention relates to a population of Treg cells, characterized in that this subpopulation of Treg cells expresses at least three cytotoxic molecules. In one embodiment, this population of Treg cells has a therapeutic potential.

As used herein, the term "Treg cell population with therapeutic potential" refers to a Treg cell population whose administration to a patient induces a therapeutic response of the patient.

In one embodiment, a therapeutic response of a patient to a Treg cell therapy may correspond to an observable and/or measurable reduction in or absence of one or more of the following: reduction in the number of pathogenic cells; reduction in the percent of total cells that are pathogenic; and/or relief to some extent, of one or more of the symptoms associated with the specific disease or condition to be treated with the Treg cell therapy; reduced morbidity and mortality, and improvement in quality of life issues.

In another embodiment, the therapeutic response of a patient may correspond to an improvement of a clinical score (such as, for example, CDAI for Crohn's disease, Harvey-Bradshaw index for Crohn's disease, Mayo score (for IBD), Vitreous haze (for uveitis), DAS28 (for rheumatoid arthritis)).

In another embodiment, the therapeutic response of a patient may correspond to an improvement in quality of life issues, which may be measured, for example, using scores well known in the art, such as, for example, IBDQ for Crohn's disease or SF36 for arthritis.

In another embodiment, the therapeutic response of a patient may be assessed by imagery methods, aiming at visualizing inflammatory lesions, tissue destruction, biomarkers of autoimmunity, infiltration, edema, renewal of tissues (such as, for example, healing). For example, in the treatment of Crohn's, the therapeutic response of the patient may be assessed by endoscopy; for the treatment of uveitis, the therapeutic response of the patient may be assessed by dilated fundus examination; and the like. Examples of methods that may be used include, but are not limited to, tissue imaging with ultrasounds (especially when the disease is Rheumatoid Arthritis.), endoscopic imaging and videocapsule (especially when the disease is IBD), IRM, Scanner, contrast imaging (angiography), ophtalmoscopy, tissue tomography and the like.

In another embodiment, the therapeutic response of a patient may be assessed by measuring the level of a marker of inflammation in a sample obtained from the patient, wherein said sample may be, for example, a serum sample or a stool sample. Examples of serum markers of inflammation include, but are not limited to, blood or tissue C-Reactive Protein (CRP), pro-inflammatory cytokines (IFN-γ, IL-12, IL-23, IL-2, IL-4, IL-17, IL-18, IL-9, IL-21, IL-6, IL-8, TNF-alpha) pro-inflammatory monocytes, presence of circulating Th17, Th2 and/or Th1 lymphocytes, increased circulating leucocytes, decreased hematocrit and increased neutrophil count, circulating autoantibodies, circulating anaphylatoxins, increase circulating self-antigen or allergen specific lymphocytes, feces derived calprotectin (IBD), and the like.

In one embodiment, the at least three cytotoxic molecules comprise granzyme B.

In one embodiment, the at least three cytotoxic molecules comprise granzyme B and granzyme H.

In one embodiment, the Treg cell population of the invention expresses 3 cytotoxic molecules selected from the group comprising granulysin, granzyme A, granzyme B, granzyme M, granzyme K, granzyme H and perforin.

In one embodiment, the Treg cell population of the invention expresses granulysin, granzyme B and granzyme A.

In one embodiment, the Treg cell population of the invention expresses granulysin, granzyme B and granzyme M.

In one embodiment, the Treg cell population of the invention expresses granulysin, granzyme B and granzyme K.

In one embodiment, the Treg cell population of the invention expresses granulysin, granzyme B and granzyme H.

In one embodiment, the Treg cell population of the invention expresses granulysin, granzyme B and perforin.

In one embodiment, the Treg cell population of the invention expresses granulysin, granzyme A and granzyme M.

In one embodiment, the Treg cell population of the invention expresses granulysin, granzyme A and granzyme K.

In one embodiment, the Treg cell population of the invention expresses granulysin, granzyme A and granzyme H.

In one embodiment, the Treg cell population of the invention expresses granulysin, granzyme A and perforin.

In one embodiment, the Treg cell population of the invention expresses granulysin, granzyme M and granzyme K.

In one embodiment, the Treg cell population of the invention expresses granulysin, granzyme M and granzyme H.

In one embodiment, the Treg cell population of the invention expresses granulysin, granzyme M and perforin.

In one embodiment, the Treg cell population of the invention expresses granulysin, granzyme K and granzyme H.

In one embodiment, the Treg cell population of the invention expresses granulysin, granzyme K and perforin.

In one embodiment, the Treg cell population of the invention expresses granulysin, granzyme H and perforin.

In one embodiment, the Treg cell population of the invention expresses granzyme B, granzyme A and granzyme M.

In one embodiment, the Treg cell population of the invention expresses granzyme B, granzyme A and granzyme K.

In one embodiment, the Treg cell population of the invention expresses granzyme B, granzyme A and granzyme H.

In one embodiment, the Treg cell population of the invention expresses granzyme B, granzyme A and perforin.

In one embodiment, the Treg cell population of the invention expresses granzyme B, granzyme M and granzyme K.

In one embodiment, the Treg cell population of the invention expresses granzyme B, granzyme M and granzyme H.

In one embodiment, the Treg cell population of the invention expresses granzyme B, granzyme M and perforin.

In one embodiment, the Treg cell population of the invention expresses granzyme B, granzyme K and granzyme H.

In one embodiment, the Treg cell population of the invention expresses granzyme B, granzyme K and perforin.

In one embodiment, the Treg cell population of the invention expresses granzyme B, granzyme H and perforin.

In one embodiment, the Treg cell population of the invention expresses granzyme A, granzyme M and granzyme K.

In one embodiment, the Treg cell population of the invention expresses granzyme A, granzyme M and granzyme H.

In one embodiment, the Treg cell population of the invention expresses granzyme A, granzyme M and perforin.

In one embodiment, the Treg cell population of the invention expresses granzyme A, granzyme K and granzyme H.

In one embodiment, the Treg cell population of the invention expresses granzyme A, granzyme K and perforin.

In one embodiment, the Treg cell population of the invention expresses granzyme A, granzyme H and perforin.

In one embodiment, the Treg cell population of the invention expresses granzyme M, granzyme K and granzyme H.

In one embodiment, the Treg cell population of the invention expresses granzyme M, granzyme K and perforin.

In one embodiment, the Treg cell population of the invention expresses granzyme M, granzyme H and perforin.

In one embodiment, the Treg cell population of the invention expresses granzyme K, granzyme H and perforin.

In one embodiment, the Treg cell population of the invention expresses 4 cytotoxic molecules selected from the group comprising granulysin, granzyme A, granzyme B, granzyme M, granzyme K, granzyme H and perforin.

In one embodiment, the Treg cell population of the invention expresses granulysin, granzyme B, granzyme A and granzyme M.

In one embodiment, the Treg cell population of the invention expresses granulysin, granzyme B, granzyme A and granzyme K.

In one embodiment, the Treg cell population of the invention expresses granulysin, granzyme B, granzyme A and granzyme H.

In one embodiment, the Treg cell population of the invention expresses granulysin, granzyme B, granzyme A and perforin.

In one embodiment, the Treg cell population of the invention expresses granulysin, granzyme B, granzyme M and granzyme K.

In one embodiment, the Treg cell population of the invention expresses granulysin, granzyme B, granzyme M and granzyme H.

In one embodiment, the Treg cell population of the invention expresses granulysin, granzyme B, granzyme M and perforin.

In one embodiment, the Treg cell population of the invention expresses granulysin, granzyme B, granzyme K and granzyme H.

In one embodiment, the Treg cell population of the invention expresses granulysin, granzyme B, granzyme K and perforin.

In one embodiment, the Treg cell population of the invention expresses granulysin, granzyme B, granzyme H and perforin.

In one embodiment, the Treg cell population of the invention expresses granulysin, granzyme A, granzyme M and granzyme K.

In one embodiment, the Treg cell population of the invention expresses granulysin, granzyme A, granzyme M and granzyme H.

In one embodiment, the Treg cell population of the invention expresses granulysin, granzyme A, granzyme M and perforin.

In one embodiment, the Treg cell population of the invention expresses granulysin, granzyme A, granzyme K and granzyme H.

In one embodiment, the Treg cell population of the invention expresses granulysin, granzyme A, granzyme K and perforin.

In one embodiment, the Treg cell population of the invention expresses granulysin, granzyme A, granzyme H and perforin.

In one embodiment, the Treg cell population of the invention expresses granulysin, granzyme M, granzyme K and granzyme H.

In one embodiment, the Treg cell population of the invention expresses granulysin, granzyme M, granzyme K and perforin.

In one embodiment, the Treg cell population of the invention expresses granulysin, granzyme M, granzyme H and perforin.

In one embodiment, the Treg cell population of the invention expresses granulysin, granzyme K, granzyme H and perforin.

In one embodiment, the Treg cell population of the invention expresses granzyme B, granzyme A, granzyme M and granzyme K.

In one embodiment, the Treg cell population of the invention expresses granzyme B, granzyme A, granzyme M and granzyme H.

In one embodiment, the Treg cell population of the invention expresses granzyme B, granzyme A, granzyme M and perforin.

In one embodiment, the Treg cell population of the invention expresses granzyme B, granzyme A, granzyme K and granzyme H.

In one embodiment, the Treg cell population of the invention expresses granzyme B, granzyme A, granzyme K and perforin.

In one embodiment, the Treg cell population of the invention expresses granzyme B, granzyme A, granzyme H and perforin.

In one embodiment, the Treg cell population of the invention expresses granzyme B, granzyme M, granzyme K and granzyme H.

In one embodiment, the Treg cell population of the invention expresses granzyme B, granzyme M, granzyme K and perforin.

In one embodiment, the Treg cell population of the invention expresses granzyme B, granzyme M, granzyme H and perforin.

In one embodiment, the Treg cell population of the invention expresses granzyme B, granzyme K, granzyme H and perforin.

In one embodiment, the Treg cell population of the invention expresses granzyme A, granzyme M, granzyme K and granzyme H.

In one embodiment, the Treg cell population of the invention expresses granzyme A, granzyme M, granzyme K and perforin.

In one embodiment, the Treg cell population of the invention expresses granzyme A, granzyme M, granzyme H and perforin.

In one embodiment, the Treg cell population of the invention expresses granzyme A, granzyme K, granzyme H and perforin.

In one embodiment, the Treg cell population of the invention expresses granzyme M, granzyme K, granzyme H and perforin.

In one embodiment, the Treg cell population of the invention expresses 5 cytotoxic molecules selected from the group comprising granulysin, granzyme A, granzyme B, granzyme M, granzyme K, granzyme H and perforin.

In one embodiment, the Treg cell population of the invention expresses granulysin, granzyme B, granzyme A, granzyme M and granzyme K.

In one embodiment, the Treg cell population of the invention expresses granulysin, granzyme B, granzyme A, granzyme M and granzyme H.

In one embodiment, the Treg cell population of the invention expresses granulysin, granzyme B, granzyme A, granzyme M and perforin.

In one embodiment, the Treg cell population of the invention expresses granulysin, granzyme B, granzyme A, granzyme K and granzyme H.

In one embodiment, the Treg cell population of the invention expresses granulysin, granzyme B, granzyme A, granzyme K and perforin.

In one embodiment, the Treg cell population of the invention expresses granulysin, granzyme B, granzyme M, granzyme K and granzyme H.

In one embodiment, the Treg cell population of the invention expresses granulysin, granzyme B, granzyme M, granzyme K and perforin.

In one embodiment, the Treg cell population of the invention expresses granulysin, granzyme B, granzyme M, granzyme H and perforin.

In one embodiment, the Treg cell population of the invention expresses granulysin, granzyme B, granzyme K, granzyme H and perforin.

In one embodiment, the Treg cell population of the invention expresses granulysin, granzyme B, granzyme A, granzyme H and perforin.

In one embodiment, the Treg cell population of the invention expresses granulysin, granzyme A, granzyme M, granzyme K and granzyme H.

In one embodiment, the Treg cell population of the invention expresses granulysin, granzyme A, granzyme M, granzyme K and perforin.

In one embodiment, the Treg cell population of the invention expresses granulysin, granzyme A, granzyme M, granzyme H and perforin.

In one embodiment, the Treg cell population of the invention expresses granulysin, granzyme A, granzyme K, granzyme H and perforin.

In one embodiment, the Treg cell population of the invention expresses granulysin, granzyme M, granzyme K, granzyme H and perforin.

In one embodiment, the Treg cell population of the invention expresses granzyme B, granzyme A, granzyme M, granzyme K and granzyme H.

In one embodiment, the Treg cell population of the invention expresses granzyme B, granzyme A, granzyme K, granzyme H and perforin.

In one embodiment, the Treg cell population of the invention expresses granzyme B, granzyme A, granzyme M, granzyme H and perforin.

In one embodiment, the Treg cell population of the invention expresses granzyme B, granzyme A, granzyme M, granzyme K and perforin.

In one embodiment, the Treg cell population of the invention expresses granzyme B granzyme M, granzyme K, granzyme H and perforin.

In one embodiment, the Treg cell population of the invention expresses granzyme A, granzyme M, granzyme K, granzyme H and perforin.

In one embodiment, the Treg cell population of the invention expresses 6 cytotoxic moelcules selected from the group comprising granulysin, granzyme A, granzyme B, granzyme M, granzyme K, granzyme H and perforin.

In one embodiment, the Treg cell population of the invention expresses granulysin, granzyme B, granzyme A, granzyme M, granzyme K and granzyme H.

In one embodiment, the Treg cell population of the invention expresses granulysin, granzyme B, granzyme A, granzyme M, granzyme K and perforin.

In one embodiment, the Treg cell population of the invention expresses granulysin, granzyme B, granzyme A, granzyme M, granzyme H and perforin.

In one embodiment, the Treg cell population of the invention expresses granulysin, granzyme B, granzyme A, granzyme K, granzyme H and perforin.

In one embodiment, the Treg cell population of the invention expresses granulysin, granzyme B, granzyme M, granzyme K, granzyme H and perforin.

In one embodiment, the Treg cell population of the invention expresses granulysin, granzyme A, granzyme M, granzyme K, granzyme H and perforin.

In one embodiment, the Treg cell population of the invention expresses granzyme B, granzyme A, granzyme M, granzyme K, granzyme H and perforin.

In one embodiment, the Treg cell population of the invention expresses granulysin, granzyme A, granzyme B, granzyme M, granzyme K, granzyme H and perforin.

The terms "expressing (or +)" and "not expressing (or -)" are well known in the art and refer to the expression level of the cell marker of interest, in that the expression level of the cell marker corresponding to "+" is high or intermediate, also referred as "+/-", and the expression level of the cell marker corresponding to "-" is null.

The expression level of the cell marker of interest is determined by comparing the fluorescence intensity (FI) of the cells from the Treg cell population stained with fluorescently labeled antibody specific for this marker to the FI of the cells from the same Treg cell population stained with fluorescently labeled antibody with an irrelevant specificity but with the same isotype, the same fluorescent probe and originated from the same specie (referred as Isotype control). The cells from the Treg population stained with fluorescently labeled antibody specific for this marker and that show equivalent FI or a lower FI than the cells stained with the isotype controls are not expressing this marker and then are designated (-) or negative. The cells from the Treg population stained with fluorescently labeled antibody specific for this marker and that show a FI value superior to the cells stained with the isotype controls are expressing this markers and then are designated (+) or positive.

In one embodiment, at least 30% of the cells of the Treg cell population of the invention express said at least 3 cytotoxic molecules.

In one embodiment, the Treg cell population of the invention is such that
- at least about 30%, preferably at least about 40, 50, 60, 70, 80% or more of the cells of the Treg population express granzyme B,
- at least about 80%, preferably at least about 85, 90% or more of the cells of the Treg population express granzyme H, and
- at least about 30%, preferably at least about 40, 50, 60, 70, 80% or more of the cells of the Treg population express at least one cytotoxic molecule selected from granulysin, granzyme A, granzyme K, granzyme M and perforin.

In one embodiment, the Treg cell population of the invention is characterized in that the score resulting from the sum of:
- the percentage of cells of the Treg cell population expressing a first cytotoxic molecule, and
- the percentage of cells of the Treg cell population expressing a second cytotoxic molecule,
- the percentage of cells of the Treg cell population expressing a third cytotoxic molecule,
- optionally the percentage of cells of the Treg cell population expressing a fourth cytotoxic molecule,
- optionally the percentage of cells of the Treg cell population expressing a fifth cytotoxic molecule,
- optionally the percentage of cells of the Treg cell population expressing a sixth cytotoxic molecule, and
- optionally the percentage of cells of the Treg cell population expressing a seventh cytotoxic molecule,
is superior to, a reference score measured in Treg cell populations that do not induce a therapeutic response when used in a Treg cell therapy in a patient.

In one embodiment, the Treg cell population of the invention is characterized in that the score resulting from the sum of:
- the percentage of cells of the Treg cell population expressing a first cytotoxic molecule, and
- the percentage of cells of the Treg cell population expressing a second cytotoxic molecule,
- the percentage of cells of the Treg cell population expressing a third cytotoxic molecule,
- optionally the percentage of cells of the Treg cell population expressing a fourth cytotoxic molecule,
- optionally the percentage of cells of the Treg cell population expressing a fifth cytotoxic molecule,
- optionally the percentage of cells of the Treg cell population expressing a sixth cytotoxic molecule, and
- optionally the percentage of cells of the Treg cell population expressing a seventh cytotoxic molecule
is equivalent or superior to a reference score measured in Treg cell populations that induce a therapeutic response when used in a Treg cell therapy in a patient.

In one embodiment, the Treg cell population with therapeutic potential of the invention is an engineered Treg cell population with therapeutic potential.

In one embodiment, an engineered Treg cell is obtained by transduction of at least three nucleic acid sequences encoding at least three cytotoxic molecules, wherein said at least three cytotoxic molecules are selected from granulysin, granzyme A, granzyme B, granzyme M, granzyme K, granzyme H and perforin. In one embodiment, the at least three cytotoxic molecules are transduced in an isolated Treg cell, thereby obtaining an engineered Treg cell with therapeutic potential.

In another embodiment, the at least three cytotoxic molecules are transduced in an naïve T cell, and the transduced T cell is then in vitro differentiated, thereby obtaining an engineered Treg cell with therapeutic potential.

In still another embodiment, a naïve T cell is in vitro differentiated, thereby obtaining a Treg cell, and then the at least three cytotoxic molecules are transduced in the in vitro differentiated Treg cell, thereby obtaining an engineered Treg cell with therapeutic potential.

Method for in vitro differentiating T cells within Treg cells are well known from the skilled artisan.

In another embodiment, engineered Treg cells are obtained by transducing T cells with at least one gene such as for example FoxP3 or IL-10 as it is well known in the art. Then the engineered Treg cells are optionnaly further transduced with the at least three cytotoxic molecules to obtain the Treg cell population of the invention.

The present invention also relates to a method for obtaining an engineered Treg cell population with therapeutic potential, wherein said method comprises a step of transduction of a cell with at least three nucleic acid sequences encoding at least three cytotoxic molecules, wherein said at least three cytotoxic molecules are selected from granulysin, granzyme A, granzyme B, granzyme M, granzyme K, granzyme H and perforin.

In one embodiment, the transduction step is carried out on an isolated Treg cell, on a naive T cell, on an in vitro differentiated Treg cell or on an engineered Treg cell.

In one embodiment, the Treg cell population of the invention is a Tr1 cell population.

In one embodiment, the Treg cell population of the invention is antigen-specific.

In one embodiment, the Treg cell population is a Treg clone, preferably an antigen-specific Treg clone.

In one embodiment, the Treg cell population is a human Treg cell population.

In one embodiment, the Treg cell population is an isolated Treg cell population.

The present invention also relates to a pharmaceutical composition comprising at least one Treg cell population as described hereinabove, and at least one pharmaceutically acceptable excipient.

As used herein, the term "excipient" refers to any and all solvents, dispersion media, coatings, antibacterial and antifungal agents, isotonic and absorption delaying agents and the like. In one embodiment, the excipient comprises human serum albumin. For human administration, preparations should meet sterility, pyrogenicity, general safety and purity standards as required by regulatory offices, such as, for example, FDA Office or EMA.

By "pharmaceutically acceptable" is meant that the ingredients of a pharmaceutical composition are compatible with each other and not deleterious to the subject to which it is administered.

The present invention also relates to a medicament comprising a Treg cell population as described hereinabove.

The present invention also relates to a method for identifying Treg cells with therapeutic potential, wherein said method comprises determining the expression level of cytotoxic molecules, wherein said expression level of cytotoxic molecules comprises the expression level of at least 3 cytotoxic molecules selected from the group comprising granulysin, granzyme A, granzyme B, granzyme M, granzyme K, granzyme H and perforin, wherein the Treg cells expressing at least three cytotoxic molecules are Treg cells with therapeutic potential.

In one embodiment, the at least three cytotoxic molecules comprise granzyme B.

In one embodiment, the at least three cytotoxic molecules comprise granzyme B and granzyme H.

In one embodiment, the method of the invention comprises determining the expression level of 3 cytotoxic molecules selected from the group comprising granulysin, granzyme A, granzyme B, granzyme M, granzyme K, granzyme H and perforin.

In one embodiment, the method of the invention comprises determining the expression level of granulysin, granzyme B and granzyme A.

In one embodiment, the method of the invention comprises determining the expression level of granulysin, granzyme B and granzyme M.

In one embodiment, the method of the invention comprises determining the expression level of granulysin, granzyme B and granzyme K.

In one embodiment, the method of the invention comprises determining the expression level of granulysin, granzyme B and granzyme H.

In one embodiment, the method of the invention comprises determining the expression level of granulysin, granzyme B and perforin.

In one embodiment, the method of the invention comprises determining the expression level of granulysin, granzyme A and granzyme M.

In one embodiment, the method of the invention comprises determining the expression level of granulysin, granzyme A and granzyme K.

In one embodiment, the method of the invention comprises determining the expression level of granulysin, granzyme A and granzyme H.

In one embodiment, the method of the invention comprises determining the expression level of granulysin, granzyme A and perforin.

In one embodiment, the method of the invention comprises determining the expression level of granulysin, granzyme M and granzyme K.

In one embodiment, the method of the invention comprises determining the expression level of granulysin, granzyme M and granzyme H.

In one embodiment, the method of the invention comprises determining the expression level of granulysin, granzyme M and perforin.

In one embodiment, the method of the invention comprises determining the expression level of granulysin, granzyme K and granzyme H.

In one embodiment, the method of the invention comprises determining the expression level of granulysin, granzyme K and perforin.

In one embodiment, the method of the invention comprises determining the expression level of granulysin, granzyme H and perforin.

In one embodiment, the method of the invention comprises determining the expression level of granzyme B, granzyme A and granzyme M.

In one embodiment, the method of the invention comprises determining the expression level of granzyme B, granzyme A and granzyme K.

In one embodiment, the method of the invention comprises determining the expression level of granzyme B, granzyme A and granzyme H.

In one embodiment, the method of the invention comprises determining the expression level of granzyme B, granzyme A and perforin.

In one embodiment, the method of the invention comprises determining the expression level of granzyme B, granzyme M and granzyme K.

In one embodiment, the method of the invention comprises determining the expression level of granzyme B, granzyme M and granzyme H.

In one embodiment, the method of the invention comprises determining the expression level of granzyme B, granzyme M and perforin.

In one embodiment, the method of the invention comprises determining the expression level of granzyme B, granzyme K and granzyme H.

In one embodiment, the method of the invention comprises determining the expression level of granzyme B, granzyme K and perforin.

In one embodiment, the method of the invention comprises determining the expression level of granzyme B, granzyme H and perforin.

In one embodiment, the method of the invention comprises determining the expression level of granzyme A, granzyme M and granzyme K.

In one embodiment, the method of the invention comprises determining the expression level of granzyme A, granzyme M and granzyme H.

In one embodiment, the method of the invention comprises determining the expression level of granzyme A, granzyme M and perforin.

In one embodiment, the method of the invention comprises determining the expression level of granzyme A, granzyme K and granzyme H.

In one embodiment, the method of the invention comprises determining the expression level of granzyme A, granzyme K and perforin.

In one embodiment, the method of the invention comprises determining the expression level of granzyme A, granzyme H and perforin.

In one embodiment, the method of the invention comprises determining the expression level of granzyme M, granzyme K and granzyme H.

In one embodiment, the method of the invention comprises determining the expression level of granzyme M, granzyme K and perforin.

In one embodiment, the method of the invention comprises determining the expression level of granzyme M, granzyme H and perforin.

In one embodiment, the method of the invention comprises determining the expression level of granzyme K, granzyme H and perforin.

In one embodiment, the method of the invention comprises determining the expression level of 4 cytotoxic molecules selected from the group comprising granulysin, granzyme A, granzyme B, granzyme M, granzyme K, granzyme H and perforin.

In one embodiment, the method of the invention comprises determining the expression level of granulysin, granzyme B, granzyme A and granzyme M.

In one embodiment, the method of the invention comprises determining the expression level of granulysin, granzyme B, granzyme A and granzyme K.

In one embodiment, the method of the invention comprises determining the expression level of granulysin, granzyme B, granzyme A and granzyme H.

In one embodiment, the method of the invention comprises determining the expression level of granulysin, granzyme B, granzyme A and perforin.

In one embodiment, the method of the invention comprises determining the expression level of granulysin, granzyme B, granzyme M and granzyme K.

In one embodiment, the method of the invention comprises determining the expression level of granulysin, granzyme B, granzyme M and granzyme H.

In one embodiment, the method of the invention comprises determining the expression level of granulysin, granzyme B, granzyme M and perforin.

In one embodiment, the method of the invention comprises determining the expression level of granulysin, granzyme B, granzyme K and granzyme H.

In one embodiment, the method of the invention comprises determining the expression level of granulysin, granzyme B, granzyme K and perforin.

In one embodiment, the method of the invention comprises determining the expression level of granulysin, granzyme B, granzyme H and perforin.

In one embodiment, the method of the invention comprises determining the expression level of granulysin, granzyme A, granzyme M and granzyme K.

In one embodiment, the method of the invention comprises determining the expression level of granulysin, granzyme A, granzyme M and granzyme H.

In one embodiment, the method of the invention comprises determining the expression level of granulysin, granzyme A, granzyme M and perforin.

In one embodiment, the method of the invention comprises determining the expression level of granulysin, granzyme A, granzyme K and granzyme H.

In one embodiment, the method of the invention comprises determining the expression level of granulysin, granzyme A, granzyme K and perforin.

In one embodiment, the method of the invention comprises determining the expression level of granulysin, granzyme A, granzyme H and perforin.

In one embodiment, the method of the invention comprises determining the expression level of granulysin, granzyme M, granzyme K and granzyme H.

In one embodiment, the method of the invention comprises determining the expression level of granulysin, granzyme M, granzyme K and perforin.

In one embodiment, the method of the invention comprises determining the expression level of granulysin, granzyme M, granzyme H and perforin.

In one embodiment, the method of the invention comprises determining the expression level of granulysin, granzyme K, granzyme H and perforin.

In one embodiment, the method of the invention comprises determining the expression level of granzyme B, granzyme A, granzyme M and granzyme K.

In one embodiment, the method of the invention comprises determining the expression level of granzyme B, granzyme A, granzyme M and granzyme H.

In one embodiment, the method of the invention comprises determining the expression level of granzyme B, granzyme A, granzyme M and perforin.

In one embodiment, the method of the invention comprises determining the expression level of granzyme B, granzyme A, granzyme K and granzyme H.

In one embodiment, the method of the invention comprises determining the expression level of granzyme B, granzyme A, granzyme K and perforin.

In one embodiment, the method of the invention comprises determining the expression level of granzyme B, granzyme A, granzyme HP and granzyme.

In one embodiment, the method of the invention comprises determining the expression level of granzyme B, granzyme M, granzyme K and granzyme H.

In one embodiment, the method of the invention comprises determining the expression level of granzyme B, granzyme M, granzyme K and perforin.

In one embodiment, the method of the invention comprises determining the expression level of granzyme B, granzyme M, granzyme H and perforin.

In one embodiment, the method of the invention comprises determining the expression level of granzyme B, granzyme K, granzyme H and perforin.

In one embodiment, the method of the invention comprises determining the expression level of granzyme A, granzyme M, granzyme K and granzyme H.

In one embodiment, the method of the invention comprises determining the expression level of granzyme A, granzyme M, granzyme K and perforin.

In one embodiment, the method of the invention comprises determining the expression level of granzyme A, granzyme M, granzyme H and perforin.

In one embodiment, the method of the invention comprises determining the expression level of granzyme A, granzyme K, granzyme H and perforin.

In one embodiment, the method of the invention comprises determining the expression level of granzyme M, granzyme K, granzyme H and perforin.

In one embodiment, the method of the invention comprises determining the expression level of 5 cytotoxic molecules selected from the group comprising granulysin, granzyme A, granzyme B, granzyme M, granzyme K, granzyme H and perforin.

In one embodiment, the method of the invention comprises determining the expression level of granulysin, granzyme B, granzyme A, granzyme M and granzyme K.

In one embodiment, the method of the invention comprises determining the expression level of granulysin, granzyme B, granzyme A, granzyme M and granzyme H.

In one embodiment, the method of the invention comprises determining the expression level of granulysin, granzyme B, granzyme A, granzyme M and perforin.

In one embodiment, the method of the invention comprises determining the expression level of granulysin, granzyme B, granzyme A, granzyme K and granzyme H.

In one embodiment, the method of the invention comprises determining the expression level of granulysin, granzyme B, granzyme A, granzyme K and perforin.

In one embodiment, the method of the invention comprises determining the expression level of granulysin, granzyme B, granzyme M, granzyme K and granzyme H.

In one embodiment, the method of the invention comprises determining the expression level of granulysin, granzyme B, granzyme M, granzyme K and perforin.

In one embodiment, the method of the invention comprises determining the expression level of granulysin, granzyme B, granzyme M, granzyme H and perforin.

In one embodiment, the method of the invention comprises determining the expression level of granulysin, granzyme B, granzyme K, granzyme H and perforin.

In one embodiment, the method of the invention comprises determining the expression level of granulysin, granzyme B, granzyme A, granzyme H and perforin.

In one embodiment, the method of the invention comprises determining the expression level of granulysin, granzyme A, granzyme M, granzyme K and granzyme H.

In one embodiment, the method of the invention comprises determining the expression level of granulysin, granzyme A, granzyme M, granzyme K and perforin.

In one embodiment, the method of the invention comprises determining the expression level of granulysin, granzyme A, granzyme M, granzyme H and perforin.

In one embodiment, the method of the invention comprises determining the expression level of granulysin, granzyme A, granzyme K, granzyme H and perforin.

In one embodiment, the method of the invention comprises determining the expression level of granulysin, granzyme M, granzyme K, granzyme H and perforin.

In one embodiment, the method of the invention comprises determining the expression level of granzyme B, granzyme A, granzyme M, granzyme K and granzyme H.

In one embodiment, the method of the invention comprises determining the expression level of granzyme B, granzyme A, granzyme K, granzyme H and perforin.

In one embodiment, the method of the invention comprises determining the expression level of granzyme B, granzyme A, granzyme M, granzyme H and perforin.

In one embodiment, the method of the invention comprises determining the expression level of granzyme B, granzyme A, granzyme M, granzyme K and perforin.

In one embodiment, the method of the invention comprises determining the expression level of granzyme B granzyme M, granzyme K, granzyme H and perforin.

In one embodiment, the method of the invention comprises determining the expression level of granzyme A, granzyme M, granzyme K, granzyme H and perforin.

In one embodiment, the method of the invention comprises determining the expression level of 6 cytotoxic molecules selected from the group comprising granulysin, granzyme A, granzyme B, granzyme M, granzyme K, granzyme H and perforin.

In one embodiment, the method of the invention comprises determining the expression level of granulysin, granzyme B, granzyme A, granzyme M, granzyme K and granzyme H.

In one embodiment, the method of the invention comprises determining the expression level of granulysin, granzyme B, granzyme A, granzyme M, granzyme K and perforin. In one embodiment, the method of the invention comprises determining the expression level of granulysin, granzyme B, granzyme A, granzyme M, granzyme H and perforin. In one embodiment, the method of the invention comprises determining the expression level of granulysin, granzyme B, granzyme A, granzyme K, granzyme H and perforin. In one embodiment, the method of the invention comprises determining the expression level of granulysin, granzyme B, granzyme M, granzyme K, granzyme H and perforin. In one embodiment, the method of the invention comprises determining the expression level of granulysin, granzyme A, granzyme M, granzyme K, granzyme H and perforin. In one embodiment, the method of the invention comprises determining the expression level of granzyme B, granzyme A, granzyme M, granzyme K, granzyme H and perforin.

In one embodiment, the method of the invention comprises determining the expression level of granulysin, granzyme A, granzyme B, granzyme M, granzyme K, granzyme H and perforin.

In one embodiment of the invention, a Treg cell population with therapeutic potential comprises:
- more than about 30%, preferably more than about 40, 50, 60, 70, 80% or more of cells expressing granzyme B
- more than 80%, preferably more than about 85, 90% or more of cells expressing granzyme H, and
- more than 30%, preferably more than about 40, 50, 60, 70, 80% or more of cells expressing at least one of granulysin, granzyme A, granzyme M, granzyme K and perforin.

In one embodiment, the method of the invention comprises:
a. determining in the Treg cell population:
   - the percentage of cells expressing a first cytotoxic molecule,
   - the percentage of cells expressing a second cytotoxic molecule,
   - the percentage of cells expressing a third cytotoxic molecule,
   - optionally the percentage of cells expressing a fourth cytotoxic molecule,
   - optionally the percentage of cells expressing a fifth cytotoxic molecule, and
   - optionally the percentage of cells expressing a sixth cytotoxic molecule,
   - optionally the percentage of cells expressing a seventh cytotoxic molecule,
b. summing the at least three percentages measured in step (a), thereby obtaining a score, and
c. comparing said score with a reference score.

In one embodiment, the method of the invention comprises:
a. determining in the Treg cell population:
   - the percentage of cells expressing granulysin,
   - the percentage of cells expressing granzyme B,
   - the percentage of cells expressing granzyme A,
   - the percentage of cells expressing granzyme M,
   - the percentage of cells expressing granzyme K,
   - the percentage of cells expressing granzyme H, and
   - the percentage of cells expressing perforin,
b. summing the seven percentages measured in step (a), thereby obtaining a score named "cytotoxic score", and
c. comparing said cytotoxic score with a reference cytotoxic score.

The present invention also relates to a method for selecting Treg cells with therapeutic potential, wherein said method comprises
- identifying Treg cells with therapeutic potential according to the method for identifying Treg cells with therapeutic potential described hereinabove, and
- selecting said cells.

As used herein, the term "expression" may refer alternatively to the transcription of a cytotoxic molecule (i.e. expression of the mRNA) or to the translation (i.e. expression of the protein) of a cytotoxic molecule. In one embodiment, detecting the expression may correspond to an intracellular detection. In another embodiment, detecting the expression may correspond to an extracellular detection, i.e. to the detection of secretion. In another embodiment, detecting the expression may correspond to both intracellular and extracellular detections.

Methods for determining the expression level are well-known from the skilled artisan, and include, without limitation, determining the transcriptome (in an embodiment wherein expression relates to transcription of a cytotoxic molecule) or proteome (in an embodiment wherein expression relates to translation of a cytotoxic molecule) of cells of a Treg cell population.

In one embodiment of the invention, the expression of the cytotoxic molecules is assessed at the mRNA level. Methods for assessing the transcription level of a cytotoxic molecule are well known in the prior art. Examples of such methods include, but are not limited to, RT-PCR, RT-qPCR, Northern Blot, hybridization techniques such as, for example, use of microarrays, and combination thereof including but not limited to, hybridization of amplicons obtained by RT-PCR, sequencing such as, for example, next-generation DNA sequencing (NGS) or RNA-seq (also known as "Whole Transcriptome Shotgun Sequencing") and the like.

In one embodiment of the invention, the expression of the cytotoxic molecules is assessed at the protein level. Methods for determining a protein level in a sample are well-known in the art. Examples of such methods include, but are not limited to, immunohistochemistry, Multiplex methods (Luminex), western blot, enzyme-linked immunosorbent assay (ELISA), sandwich ELISA, fluorescent-linked immunosorbent assay (FLISA), enzyme immunoassay (EIA), radioimmunoassay (RIA), flow cytometry (FACS) and the like.

In one embodiment, determining the expression level of at least one cytotoxic molecule corresponds to detecting and/or quantifying binding of a ligand to a cytotoxic molecule. In one embodiment, said ligand is an antibody specific of said cytotoxic molecule, and the method of the invention comprises detecting and/or quantifying a complex formed between said antibody and said cytotoxic molecule. The expression "detecting and/or quantifying binding of a ligand to a cytotoxic molecule" means that when a cytotoxic molecule is present a complex is formed between the cytotoxic molecule and the ligand. That complex can be detected if the ligand has been for example, but not limited to, covalently coupled with a detectable molecule such as an antibody constant fragment (Fc) or a fluorescent compound (e.g. Cyanine dye, Alexa dye, Quantum dye, etc). The complex can also be detected if the ligand has been tagged with different means well known to the person skilled in the art. For example, but without limitation, a tag used with the invention can be a tag selected from the group comprising or consisting of Hemaglutinin Tag, Poly Arginine Tag, Poly Histidine Tage, Myc Tag, Strep Tag, S-Tag, HAT Tag, 3x Flag Tag, Calmodulin-binding peptide Tag, SBP Tag, Chitin binding domain Tag, GST Tag, Maltose-Binding protein Tag, Fluorescent Protein Tag, T7 Tag, V5 Tag and Xpress Tag. The use of the ligand therefore allows on the one hand the identification and detection of the cytotoxic molecule depending on the ligand used, and on the other hand the quantification of the complex formed.

In one embodiment, determining the expression level of cytotoxic molecules is conducted by flow cytometry, immunofluorescence or image analysis, for example high content analysis. Preferably, the determination of the expression level of cytotoxic molecules is conducted by flow cytometry. In one embodiment, before conducting flow cytometry analysis, cells are fixed and permeabilized, thereby allowing detecting intracellular proteins.

In one embodiment, determining the expression level of cytotoxic molecules is conducted by one step flow cytometry, i.e. the expression level of the at least three cytotoxic molecules selected from the group comprising granulysin, granzyme B, granzyme A, granzyme M, granzyme K, granzyme H and perforin are concomitantly determined by flow cytometry, using a mix of at least three antibodies, wherein each antibody is fluorescently labelled. In one embodiment, each antibody of the mix is differently labelled (i.e. the label of the antibody binding the first cytotoxic molecule is different from the label of the antibody binding the second cytotoxic molecule...), thereby allowing determining independently the expression level of each cytotoxic molecule. In another embodiment, the same fluorescent label is used for labelling at least two antibodies of the mix (i.e. the label of the antibody binding the first cytotoxic molecule is the same than the label of the antibody binding the second cytotoxic molecule...), thereby allowing determining a combined expression level of at least two cytotoxic molecules. In another embodiment, all antibodies of the mix are labelled with the same fluorescent label, thereby allowing determining a combined expression level of cytotoxic molecules.

Examples of antibodies that may be used for detecting the cytotoxic molecules of the invention include, but are not limited to, the antibodies listed in the Table 1 below:

**Table 1**

| **Cytotoxic molecules** | **Supplier** | **Clone** |
|---|---|---|
| GranzymeA | Becton Dickinson | CB9 |
| GranzymeB | Becton Dickinson | GB11 |
| Granulysin | Becton Dickinson | RB1 |
| Perforin | Miltenyi Biotech | deltaG9 |
| GranzymeH | Interchim(sinobiological) | 03 |
| GranzymeK | eBioscience | G3H69 |
| GranzymeM | Clinisciences(LSBio) | polyclonal |

In one embodiment, determining the expression level of a cytotoxic molecule in a Treg cell population comprises determining the percentage of cells of the Treg cell population expressing the cytotoxic molecule (i.e. cells "+" for the cytotoxic molecule). Preferably, said percentage of cells expressing the cytotoxic molecule is measured by FACS.

The terms "expressing (or +)" and "not expressing (or -)" are well known in the art and refer to the expression level of the cell marker of interest, in that the expression level of the cell marker corresponding to "+" is high or intermediate, also referred as "+/-", and the expression level of the cell marker corresponding to "-" is null.

The expression level of the cell marker of interest is determined by comparing the fluorescence intensity (FI) of the cells from the Treg cell population stained with fluorescently labeled antibody specific for this marker to the FI of the cells from the same Treg cell population stained with fluorescently labeled antibody with an irrelevant specificity but with the same isotype, the same fluorescent probe and originated from the same specie (referred as Isotype control). The cells from the Treg population stained with fluorescently labeled antibody specific for this marker and that show equivalent FI or a lower FI than the cells stained with the isotype controls are not expressing this marker and then are designated (-) or negative. The cells from the Treg population stained with fluorescently labeled antibody specific for this marker and that show a FI value superior to the cells stained with the isotype controls are expressing this markers and then are designated (+) or positive.

In one embodiment, the method of the invention comprises comparing the expression level of cytotoxic molecules of the Treg cell population to be injected to a patient to a reference expression level of cytotoxic molecules. In another embodiment, the method of the invention comprises calculating a score as hereinabove described, and comparing said score to a reference score.

As used herein, the term "reference" broadly encompasses any suitable reference expression level or score which may be used as a basis for comparison with respect to the measured expression level or score. In one embodiment, the reference is constructed using algorithms and/or other methods of statistical and hierarchical classification. In another aspect, the reference expression level is stored in a database to provide a stored expression level or score and the stored expression level or score is used to determine the difference in the expression level or score. The database may, for example, be stored on a computer or a server.

In one embodiment, the reference expression level or the reference score is an index value or is derived from one or more risk prediction algorithms or computed indices for the therapeutic response to a Treg cell therapy. A reference expression level or a reference score can be relative to a number or value derived from population studies, including without limitation, such Treg cell populations isolated from subjects having similar age range, subjects in the same or similar ethnic group, subjects having family histories of chronic inflammatory diseases, autoimmune diseases, or allergic diseases.

In one embodiment of the invention, the reference expression level or the reference score is the expression level or the score (respectively) measured in one or more Treg cell populations whose injection to patients has resulted in the therapeutic response of said patients. According to this embodiment, an equivalence (i.e. an absence of difference) between the measured expression level and the reference expression level, or between the measured score and the reference score is indicative of the therapeutic potential of a Treg cell population, i.e. the administration of said Treg cell population to a patient is predicted as resulting to the therapeutic response of the patient.

In one embodiment of the invention, the reference expression level or the reference score is the expression level or the score (respectively) measured in one or more Treg cell populations whose injection to patients has resulted in the absence of therapeutic response of said patients. According to this embodiment, a difference between the measured expression level and the reference expression level, or between the measured score and the reference score is indicative of the therapeutic potential of a Treg cell population, i.e. the administration of said Treg cell population to a patient is predicted as resulting to the therapeutic response of the patient.

In one embodiment, two numeric values, in particular two expression levels or two scores, are considered as different if the first numeric value is higher (such as, for example, the first numeric value is about 5% higher than the second one, preferably is about 10, 20, 30, 40, 50, 60, 70, 80, 90% or more higher than the second one) or lower than the second one (such as, for example, the second numeric value is about 5% lower than the second one, preferably is about 10, 20, 30, 40, 50, 60, 70, 80, 90% or more lower than the second one).

The present invention also relates to a Treg cell population identified by the method for identifying Treg cells of the invention, as described hereinabove.

The present invention also relates to a Treg cell population selected by the method for selecting Treg cells of the invention, as described hereinabove.

The present invention relates to a method for predicting the efficacy of a Treg cell therapy in a patient, wherein said method comprises determining the expression level of cytotoxic molecules by the Treg cell population to be administered to the patient during the Treg cell therapy, wherein said expression level of cytotoxic molecules comprises the expression level of at least 3 cytotoxic molecules selected from the group comprising granulysin, granzyme A, granzyme B, granzyme M, granzyme K, granzyme H and perforin.

In one embodiment, the method of the invention is for evaluating the patient's risk of not responding to the treatment and that his/her condition does not improve. In another embodiment, the method of the invention is for evaluating the probability of a therapeutic response of the patient to the treatment and of an improvement of his/her condition.

In one embodiment, the method for predicting the efficacy of a Treg cell therapy in a patient or for selecting Treg cells to be used in a Treg cell therapy is performed before, preferably just before the administration to a patient, such as, for example, one year, one month or one week before the first administration of Treg cells, or less.

In one embodiment, the method for predicting the efficacy of a Treg cell therapy in a patient or for selecting Treg cells to be used in a Treg cell therapy is performed at the time of Treg product batch release.

In one embodiment, the Treg cell population was frozen before Treg cell therapy, and the method for predicting the efficacy of a Treg cell therapy in a patient or for selecting Treg cells to be used in a Treg cell therapy is performed between thawing and injection to the patient. In another embodiment, the method for predicting the efficacy of a Treg cell therapy in a patient or for selecting Treg cells to be used in a Treg cell therapy is performed before thawing.

In one embodiment of the invention, if a Treg cell population to be administered to a patient during the Treg cell therapy comprises:
- more than about 30%, preferably more than about 40, 50, 60, 70, 80% or more of cells expressing granzyme B
- more than 80%, preferably more than about 85, 90% or more of cells expressing granzyme H, and
- more than 30%, preferably more than about 40, 50, 60, 70, 80% or more of cells expressing at least one of granulysin, granzyme A, granzyme M, granzyme K and perforin,
then the patient is predicted to respond to the Treg cell therapy.

In one embodiment, the method for predicting the efficacy of a Treg cell therapy of the invention comprises:
a. determining in the Treg cell population to be administered to the subject:
   - the percentage of cells expressing a first cytotoxic molecule,
   - the percentage of cells expressing a second cytotoxic molecule,
   - the percentage of cells expressing a third cytotoxic molecule,
   - optionally the percentage of cells expressing a fourth cytotoxic molecule,
   - optionally the percentage of cells expressing a fifth cytotoxic molecule, and
   - optionally the percentage of cells expressing a sixth cytotoxic molecule,
   - optionally the percentage of cells expressing a seventh cytotoxic molecule,
b. summing the at least three percentages measured in step (a), thereby obtaining a score, and
c. comparing said score with a reference score.

In one embodiment, the method of the invention comprises:
a. determining in the Treg cell population to be administered to the subject:
   - the percentage of cells expressing granulysin,
   - the percentage of cells expressing granzyme B,
   - the percentage of cells expressing granzyme A,
   - the percentage of cells expressing granzyme M,
   - the percentage of cells expressing granzyme K,
   - the percentage of cells expressing granzyme H, and
   - the percentage of cells expressing perforin,
b. summing the seven percentages measured in step (a), thereby obtaining a score named "cytotoxic score", and
c. comparing said cytotoxic score with a reference cytotoxic score.

The present invention also relates to a method for selecting Treg cells to be used in a Treg cell therapy in a patient, comprising
a. predicting if the administration of said Treg cell population to a patient will result in a therapeutic response of said patient according to the method as described hereinabove, and
b. selecting the cells whose administration is predicted to result in a therapeutic response of said patient.

In one embodiment, the method for selecting Treg cells of the invention comprises:
a. determining the expression level of cytotoxic molecules by a Treg cell population, wherein said expression level of cytotoxic molecules comprises the expression level of at least 3 cytotoxic molecules selected from the group comprising granulysin, granzyme A, granzyme B, granzyme M, granzyme K, granzyme H and perforin,
b. comparing the expression level measured in step (a) with a reference expression level.

The present invention also relates to a Treg cell population selected by the method of selecting Treg cells to be used in a Treg cell therapy in a patient as hereinabove described.

In one embodiment, the Treg cell population with therapeutic potential is for use in a Treg cell therapy in a patient.

The present invention thus also relates to a Treg cell population with therapeutic potential as described hereinabove, or identified by the method for identifying a Treg cell population with therapeutic potential of the invention, or selected by the method for selecting a Treg cell population with therapeutic potential of the invention, for treating, for preventing or for use for preventing or treating an inflammatory condition, an autoimmune condition or for suppressing, inhibiting or preventing excessive or unwanted immune responses associated with transplantation or an allergic disease.

In one embodiment, a therapeutically effective amount of a Treg cell population with therapeutic potential as described hereinabove, or identified or selected by a method of the invention is administered to the patient. In one embodiment, the therapeutically effective amount ranges from about 10³ to about 10⁸ cells, from about 10⁴ to about 10⁷ cells, or is of about 10⁶ cells. In another embodiment, the therapeutically effective amount ranges from about 1.10² to about 5.10⁶ cells per kg of body weight of the patient, from about 1.10³ to about 5.10⁵ cells per kg of body weight of the patient, from about 1.10⁴ to 3.10⁴ cells per kg of body weight of the patient or is about 1.10⁵ cells per kg of body weight.

In one embodiment, the at least one Treg cell population is systemically administered, preferably is administered by intravenous injection or by intralymphatic injection.

In one embodiment, a single dose of the at least one Treg cell population is administered (i.e. the Treg cell therapy only comprises one administration of Treg cells). In another embodiment, the at least one Treg cell population is administered once a day, once a week, once every 2, 3, 4, 5, 6, 7 or 8 weeks or more, or once every months or every 2, 3, 4, 5, 6, 7, 8, 9, 10, 11, 12 months or more.

In one embodiment, the Treg cell therapy is an antigen-specific Treg cell therapy, i.e. comprises the administration of an antigen-specific Treg cell population. In one embodiment of the invention, the antigen-specific Treg cell population to be injected is not stimulated with said antigen prior to administration. In one embodiment of the invention, the Treg cell therapy does not comprise the administration of the antigen to which the Treg cells are specific.

In one embodiment, the antigen is a food antigen from common human diet. The term "food antigen from common human diet" refers to an immunogenic peptide, which comes from foodstuffs common for humans, such as food antigens of the following non-limiting list: bovine antigens such as lipocalin, Ca-binding S100, alpha- lactalbumin, lactoglobulins such as beta-lactoglobulin, bovine serum albumin, caseins. Food-antigens may also be atlantic salmon antigens such as parvalbumin, chicken antigens such as ovomucoid, ovalbumin, Ag22, conalbumin, lysozyme or chicken serum albumin, peanuts, shrimp antigens such as tropomyosin, wheat antigens such as agglutinin or gliadin, celery antigens such as celery profilin, carrot antigens such as carrot profilin, apple antigens such as thaumatin, apple lipid transfer protein, apple profilin, pear antigens such as pear profilin, isoflavone reductase, avocado antigens such as endochitinase, apricot antigens such as apricot lipid transfer protein, peach antigens such as peach lipid transfer protein or peach profilin, soybean antigens such as HPS, soybean profilin or (SAM22) PR-I0 prot, fragments, variants and mixtures thereof.

As used herein the term "fragment" of an antigen refers to any subset of an antigen, as a shorter peptide. In one embodiment, a fragment of an antigen is a peptide of at least 6 amino acids in length. In one embodiment, a fragment of an antigen is a peptide of 6 to 50 amino acids in length, preferably of 6 to 30 amino acids, more preferably of 6 to 20 amino acids in length.

The term "variant" of an antigen, such as, for example, a food antigen from common human diet, refers herein to an antigen that is almost identical to the natural antigen and which shares the same biological activity. The minimal difference between the natural antigen and its variants may lie for example in an amino-acid substitution, deletion, and/or addition. Such variants may contain for example conservative amino acid substitutions in which amino acid residues are replaced with amino acid residues having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

In one embodiment, the variant of an antigen presents a sequence identity of at least or about 70, 75, 80, 85, 90, 91, 92, 93, 94, 95, 96, 97, 98, 99% with the sequence of the natural antigen. The term "identity" or "identical", when used in a relationship between the sequences of two or more polypeptides, refers to the degree of sequence relatedness between polypeptides, as determined by the number of matches between strings of two or more amino acid residues. "Identity" measures the percent of identical matches between the smaller of two or more sequences with gap alignments (if any) addressed by a particular mathematical model or computer program (i.e., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988). Preferred methods for determining identity are designed to give the largest match between the sequences tested. Methods of determining identity are described in publicly available computer programs. Preferred computer program methods for determining identity between two sequences include the GCG program package, including GAP (Devereux et al., Nucl. Acid. Res. \2, 387 (1984); Genetics Computer Group, University of Wisconsin, Madison, Wis.), BLASTP, BLASTN, and FASTA (Altschul et al., J. MoI. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

In one embodiment, the antigen is an inflammatory nervous system condition -associated antigen. The term "inflammatory nervous system condition-associated antigen" refers to myelin basic protein (MBP). myelin associated glycoprotein (MAG), myelin oligodendrocyte protein (MOG), proteolipid protein (PLP), oligodendrocyte myelin oligoprotein (OMGP), myelin associated oligodendrocyte basic protein (MOBP), oligodendrocyte specific protein (OSP/Claudinl 1), heat shock proteins, oligodendrocyte specific proteins (OSP), NOGO A, glycoprotein Po, peripheral myelin protein 22 (PMP22), 2'3'-cyclic nucleotide 3'-phosphodiesterase (CNPase), fragments, variants and mixtures thereof.

In one embodiment, the antigen is a joint-associated antigen. The term "joint-associated antigen" refers to citrulline-substituted cyclic and linear filaggrin peptides, collagen type II peptides, human cartilage glycoprotein 39 (HCgp39) peptides, HSP, heterogenous nuclear ribonucleoprotein (hnRNP) A2 peptides, hnRNP Bl, hnRNP D, Ro60/52, HSP60, 65, 70 and 90, BiP, keratin, vimentin, fibrinogen, collagen type I, III, IV and V peptides, annexin V, Glucose 6 phosphate isomerase (GPI), acetyl-calpastatin, pyruvate deshydrogenase (PDH), aldolase, topoisomerase I, snRNP, PARP, Scl-70, Scl-100, phospholipid antigen including anionic cardiolipin and phosphatidylserine, neutrally charged phosphatidylethanolamine and phosphatidylcholine, matrix metalloproteinase, fibrillin, aggreccan, fragments, variants and mixtures thereof.

In one embodiment, the antigen is an eye-associated antigen. The term "eye-associated antigen" refers to type II collagen, retinal arrestin, S-arrestin, interphotoreceptor retinoid-binding proteins (IRBP1), betaB 1-crystallin, retinal proteins, choroid proteins, fragments, variants and mixtures thereof.

In one embodiment, the antigen is a human HSP antigen. The term "human HSP antigen" refers to human HSP60, HSP70, HSP90, fragments, variants and mixtures thereof.

According to one embodiment of the invention, the patient has an intestinal inflammatory condition and the Treg cells are specific of a food antigen from the common human diet.

The term "inflammatory intestinal condition" refers to inflammatory bowel disease, ulcerative colitis, Crohn's disease, intestinal inflammation linked to food allergy or intolerance, intestinal inflammation linked to milk protein allergy, intestinal inflammation linked to celiac disease, intestinal inflammation linked to hen egg allergy, or intestinal inflammation linked to peanut allergy.

In one embodiment of the invention, the patient has a Crohn's disease and the Treg cell population is ovalbumin-specific.

According to one embodiment of the invention, the patient has an inflammatory nervous system condition and the Treg cells are specific of an inflammatory nervous system condition associated-antigen.

Examples of inflammatory nervous system condition include, but are not limited to, multiple sclerosis, amyotropic lateral sclerosis (ALS), Devic's disease or NMO (neuromyelitis optica), demyelinating diseases, Alzheimer' disease (AD), Parkinson's disease (PD), poliomyelitis, motor neuron disease, (MND) Optic neuritis, Transverse myelitis, chronic inflammatory demyelinating polyneuropathy.

According to one embodiment of the invention, the patient has an arthritic condition and the Treg cells are specific of a joint-associated antigen.

The term "arthritic condition" refers to rheumatoid arthritis, polychondritis, septic arthritis, spondyloarthropathies or ankylosing spondylitis, juvenile idiopathic arthritis (JIA), psoriatic arthritis and diseases associated with arthritis such as systemic lupus erythematous, Sjogren's syndrome, scleroderma, dermatomyosotis, polymyosotis, polymyalgia rheumatica, fibromyalgia, sarcoidosis, or vasculitis.

According to one embodiment of the invention, the patient has an inflammatory condition of the eye and the Treg cells are specific of an eye-associated antigen.

Examples of "inflammatory condition of the eye" include, but are not limited to uveitis, scleritis, retinal vasculitis, diffuse unilateral subacute neuroretinitis, sympathetic ophthalmia, Vogt-Koyanagi-Harada (VKH) syndrome, sarcoidosis-related retinitis, Behçet's-related retinitis, acute retinal pigment epitheliitis and the like. Examples of uveitis include anterior uveitis (comprising iritis, iridiocyclitis, and anterior cylitis), intermediate uveitis (comprising pars planitis, posterior cyclitis, and hyalitis), posterior uveitis (comprising choroiditis, chorioretinitis, retinochoroiditis, retinitis, and neuroretinitis), panuveitis, acute uveitis, recurring uveitis and chronic uveitis.

According to one embodiment of the invention, the patient has uveitis and the Treg cells are specific of type II collagen.

According to one embodiment of the invention, the patient has an inflammatory autoimmune condition and the Treg cells are specific of a human HSP antigen.

The term "inflammatory autoimmune condition" refers to intestinal inflammatory condition such as Crohn's disease and ulcerative colitis; arthritis condition such as rheumatoid arthritis, psoriatic arthritis, ankylosing spondylitis and juvenile idiopathic arthritis; multiple sclerosis; uveitis; Wegener's disease; primary biliary cirrhosis; primary sclerosing cholangitis; asthma, transplant rejection (host versus graft disease); diabetes or graft versus host disease. More preferably, said inflammatory autoimmune disease is selected in the group of rheumatoid arthritis, Crohn's disease, multiple sclerosis, uveitis, ulcerative colitis, asthma and transplant rejection or graft versus host disease.

In one embodiment of the invention, the Treg cells may be obtained from blood, such as peripheral blood or umbilical cord blood, or from tissue biopsy such as lymph node biopsy, intestinal or synovial biopsies or mucosal tissue biopsy, or from bronchoalveolar lavage or a cerebrospinal fluid.

In one embodiment, the Treg cells have been frozen and thawed before the method of the invention is carried out.

In one embodiment, the Treg cells are autologous or allogeneic. The term "allogeneic cells" as used herein refers to cells isolated from one subject (the donor) and infused in another patient (the recipient or host). The term "autologous cells" as used herein refers to cells that are isolated and infused back into the same patient.

Examples of methods for expanding Treg cells are well known to the skilled artisan, and include, but are not limited to:
- Use of stimulatory antibodies, commonly anti-CD3 and anti-CD28, absorbed on magnetic beads or nanoparticles and use of stimulatory cytokines (commonly from the group of IL-2, IL-15 and IL-4)
- Use of stimulatory antibodies, commonly anti-CD3 and anti-CD28, and use of ligands specific for the stimulatory antibodies absorbed on magnetic beads or nanoparticles and use of stimulatory cytokines (commonly from the group of IL-2, IL-15 and IL-4)
- Use of stimulatory antibodies, commonly anti-CD3 and anti-CD28, coated on a surface of a cell culture vessel and use of stimulatory cytokines (commonly from the group of IL-2, IL-15 and IL-4), and
- use of feeder cells, as described in WO2006/108882, which is incorporated herein by reference.

Examples of methods for differentiating T cells into regulatory T cells are well known in the art and include, without limitation, exposure to rapamycin, dexamethasone, IL-10, IFN-alpha, tolerogenic antigen presenting cells such as dendritic cells and the like.

In one embodiment of the invention, regulatory T cells may be obtained by the method described in Wakkach et al (Immunity 2003 May; 18(5):605-17), and comprising the steps of:
a) isolating a progenitor cell population from a subject,
b) obtaining a population of dendritic cells by culturing said progenitor cell population in the presence of IL-10 (in a concentration ranging from 50 to 250 U/ml, preferably at 100 U/ml in the culture medium),
c) contacting cells of step b) with a CD4+ T lymphocyte population isolated from said subject in the presence of a specific antigen, to allow differentiation of CD4+ T cells directed to said antigen into the regulatory T cell population, and
d) recovering the regulatory T cell population from the step c).

Said method may also be carried out using Dexamethasone and Vitamin D3, or tolerogenised or immature DCs instead of the DCs of step b).

In another embodiment of the present invention, regulatory T cells may be obtained by the method described in the patent US6746670 and comprising the steps of:
a) culturing a CD4+ T cell population directed to a specific antigen, isolated from a subject in a media with an appropriate amount of IFN-alpha (preferably at 5 ng/ml of culture medium), and
b) recovering the regulatory T cell population.

In the step a), the media may further comprise an appropriate amount of IL-10, preferably at 100 U/ml.

In step b), the regulatory T cell population may be cultured in a media comprising IL- 15 to allow proliferation, IL- 15 being preferably at 5 ng/ml in the media.

In still another embodiment of the invention, regulatory T cells may be obtained by the method described in the patent application WO02/092793 and comprising the steps of:
a) in vitro activating a CD4+ T cell population in presence of a specific antigen, presented by artificial antigen presenting cells, and
b) recovering an activated CD4+ T cells comprising at least 10% of regulatory T cells.

Preferably, the artificial antigen presenting cells express a HLA II system molecule and a human LFA-3 molecule and do not express the co-stimulation molecules B7-1, B7-2, B7-H1, CD40, CD23 and ICAM-I.

In still another embodiment of the invention, regulatory T cells may be obtained by the method described in Groux et al. (Nature 1997, 389(6652):737-42), and comprising the steps of:
a) in vitro activating a CD4+ T cell population in presence of a specific antigen and an appropriate amount of IL-10 (preferably at 100 U/ml of culture medium); and
b) recovering the regulatory T cell population. Preferably, IL-10 is present in the media.

In still another embodiment of the invention, regulatory T cells may be obtained by the method described in the patent application WO2007/010406, comprising the steps of:
a) stimulating a leukocyte population or a peripheral blood mononuclear cell (PBMC) population with a specific antigen,
b) recovering the antigen-specific Treg cell population from the stimulated population,
c) optionally expanding said antigen-specific Treg cell population. Leukocytes encompass several types of cells, which are characterized by their importance, their distribution, their number, their lifetime and their potentiality. These types are the following : the polynuclear or granular leukocytes, among which one finds the eosinophilic, the neutrophilic and the basophilic leukocytes, and the mononuclear cells, or peripheral blood mononuclear cells (PBMCs), which are large white blood cells and consist in the major cell types of the immune system (lymphocytes and monocytes). The leukocytes or the PBMCs can be separated from the peripheral blood by any method known to those skilled in the art. Advantageously, for the separation of the PBMCs, centrifugation may be used, preferably density gradient centrifugation, preferably discontinuous density gradient centrifugation. An alternative is the use of specific monoclonal antibodies. In certain embodiments PBMC are typically isolated from the whole blood product by means of Ficoll-Hypaque, using standard procedures. In other embodiments the PBMCs are recovered by means of leukapheresis.

In still another embodiment, regulatory T cells may be obtained by:
a) culturing a leukocyte population or a peripheral blood mononuclear cell (PBMC) population with mesenchymal stem cells in the presence of a specific antigen,
b) recovering the Treg cell population.

Said method can also be carried out with naive or memory T cells instead of PBMC or leukocytes.

In another embodiment, Treg cells can be obtained by culturing T cells in the presence of IL-2 and TGF-beta (Davidson et al. The Journal of Immunology, 2007, 178:4022-4026). In another embodiment, Treg cells can be obtained by culturing T cells in the presence of TGF-beta.

In another embodiment, Treg cells can also be isolated as well-known in the art by using the following phenotype CD4+CD25+CD127- or CD49b+LAG3+.

The present invention also relates to kit for implementing the method for identifying or selecting Treg cells with therapeutic potential of the invention, or for implementing the method for predicting the therapeutic response of a patient subjected to a Treg cell therapy of the invention. In one embodiment, the kit of the invention comprises means for determining the expression level of at least three, four, five, six or seven cytotoxic molecules selected from the group comprising granulysin, granzyme A, granzyme B, granzyme M, granzyme K, granzyme H and perforin.

In one embodiment, said means for determining the expression level of at least three cytotoxic molecules are antibodies, preferably fluorescently labelled antibodies for use in flow cytometry. Examples of antibodies include the antibodies listed in the Table 1.

### BRIEF DESCRIPTION OF THE DRAWINGS

Figure 1 is a combination of graphs showing the expression of CD39 (Panel A), GITR (Panel B) and granzyme B (Panel C) by Treg cell populations injected in responder (R) or non-responder (NR) patients. Horizontal bars represent median values.
Figure 2 is a graph showing the dose dependent cytotoxicity of Treg cells on monocytes (target cells).
Figure 3 is a combination of graphs showing the effect of inhibitors on the cytotoxicity of Treg cells: (A) effect of a perforin inhibitor. (B) effect of a granzyme B inhibitor. (C) effect of broad serin protease inhibitors.
Figure 4 is a histogram showing the expression of the following cytotoxic molecules by the Treg cells of the invention. GzmB: granzyme B, GzmA: granzyme A, GzmM: granzyme M, GzmK: granzyme K, GzmH: granzyme H.
Figure 5 is a combination of graphs showing the cytotoxic score of Treg cells with therapeutic potential of the invention. (A) Cytotoxic score in Treg cell populations injected in responder (R) or non-responder (NR) patients. (B) Correlation between the cytotoxic score and the CDA score in Crohn's Disease patients.

### EXAMPLES

The present invention is further illustrated by the following examples.

### Cytotoxicity is an important mechanism of action of Treg cells

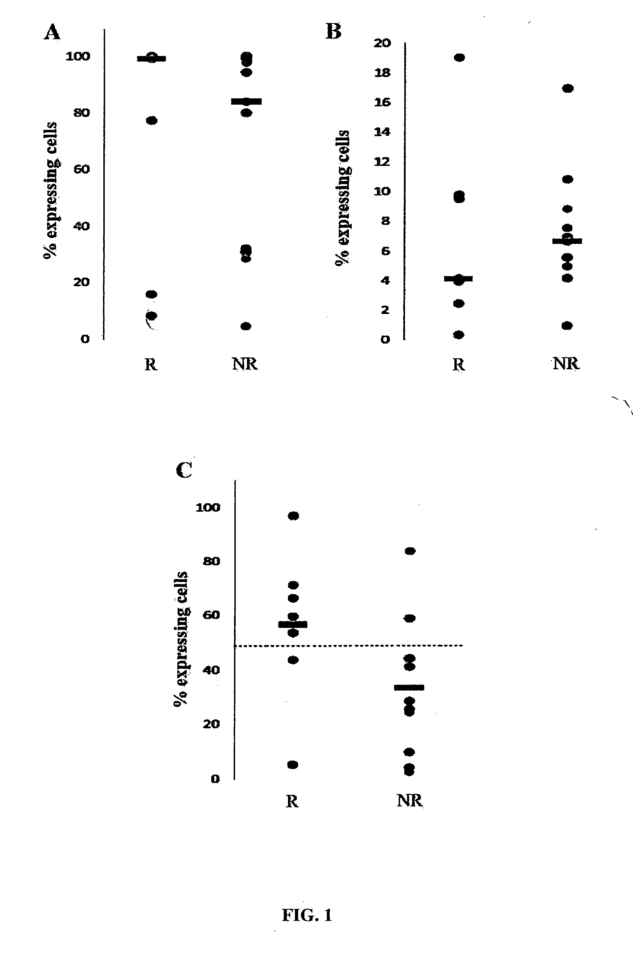

Autologous antigen-specific Treg cell populations were administered intravenously to inflammatory Crohn's Disease patients (CATS 1 Study, Gastroenterology 2012 Nov, Vol 143, 5, p 1207-1217.e2, Desreumaux P et all.). After 5 weeks, the Crohn's Disease Activity Index (CDAI) of each patient was measured. A therapeutic response was defined as a decrease of 100 points of CDAI at week 5 compared to the Day of Treg cell intravenous injection. Patients were classified as responders (R) of non-responder (NR) according to the CDAI decrease at week 5 post-injection. Expression of CD39, GITR and the cytotoxic molecule granzyme B were analyzed using flow cytometry in Treg cell populations injected in R (i.e. Treg cells with therapeutic potential) and NR patients. Results of the **Figure 1** show that the levels of CD39 and GITR are heterogeneous amongst Treg cell populations with no clear differences between R and NR patients. In contrast, Treg cell populations injected in R patients (Treg cells with therapeutic potential) expressed more granzyme B than Treg cell populations injected in NR patients. However, several Treg populations from several NR patients still expressed high levels of granzyme B (more than 50% of granzyme B expressing cells) and several Treg populations still expressed lower levels of granzyme B (less than 50% of granzyme B expressing cells).

### Cytotoxic activity of responder Treg clones

In order to characterize the cytotoxic activity of Treg cells with therapeutic potential, myeloid cells (the monocyte cell line U937) were cultured in the presence of graded amounts of Ovalbumin-specific Treg cells with therapeutic potential (Ova-Treg) at 37°C. After 4 hours of co-culture, the amount of dead cells within the monocytes was evaluated by Flow cytometry.

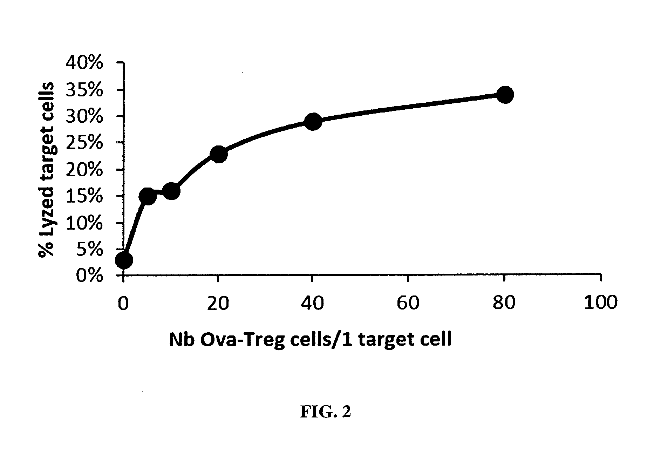

The percentage of dead cells gradually increases with the amount of Treg cells, starting from 3% in the absence of Treg cells, and up to 34% in presence of 80 Treg cells/target cells. Moreover, **Figure 2** shows that the percentage of lysed cells increases with the ratio Treg cell: monocytes.

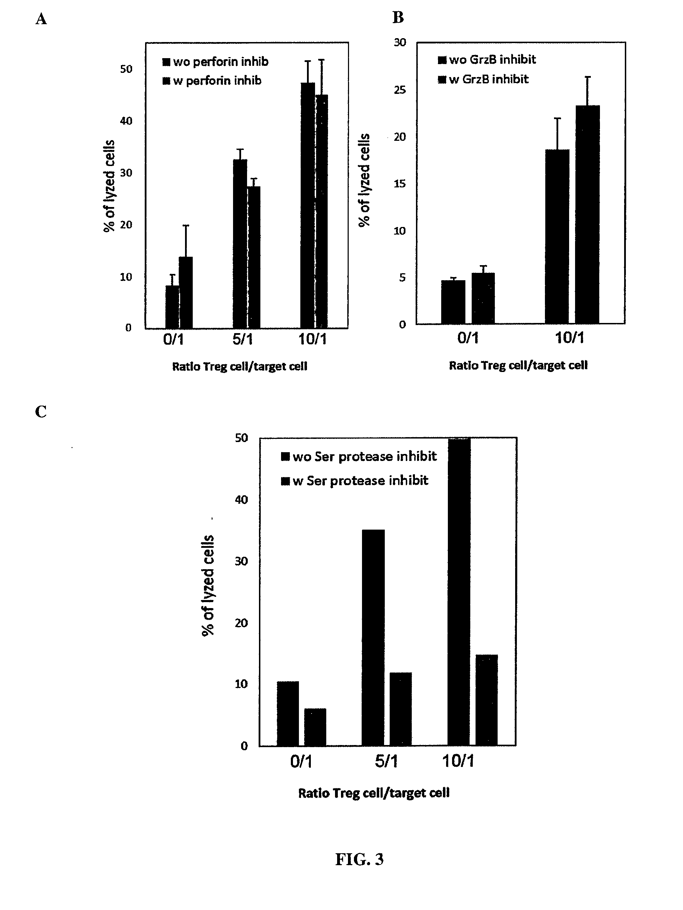

Then, myeloid cells (the monocyte cell line U937) were cultured in the presence of Ovalbumin-specific Treg cell populations (Ova-Treg) at 37°C in the presence or absence of different inhibitors of cytotoxicity: Z-AAD-CMK as granzyme B inhibitor (Calbiochem 368050), Concanamycin A as Perforin inhibitor (Sigma-Aldrich C9705-25UG) and 3,4-Dichloroisocoumarin as broad serine protease inhibitor (Sigma-Aldrich D7910-5MG). After 4 hours of co-culture, the amount of dead cells within the monocytes was evaluated by Flow cytometry. **Figure 3A** and **B** shows that cytotoxicity of Ova- Treg cells with therapeutic potential is not altered by specific perforin inhibitors or specific granzyme B inhibitors (respectively). On the contrary, **Figure 3C** shows that cytotoxicity of Ova- Treg cells with therapeutic potential is decrease by broad serine protease inhibitors. This result thus demonstrates that Treg cytotoxicity is dependent on multiple cytotoxic molecules and that perforin and granzyme B inhibition expression are not sufficient to alter the cytotoxic potential of Ova- Treg cells with therapeutic potential in vitro.

### Expression of cytotoxic molecules by regulatory T cells

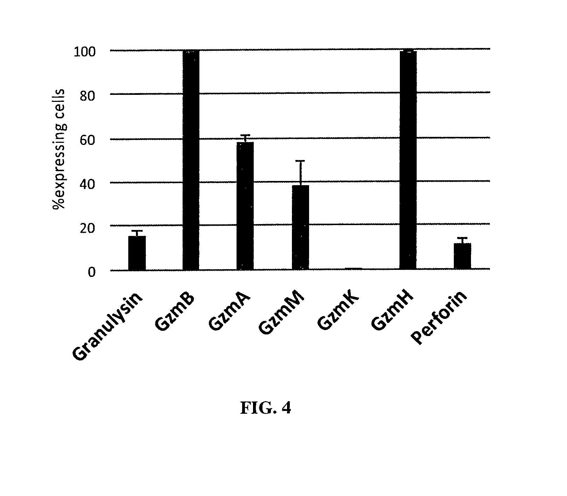

We then analyzed the expression of 7 different cytotoxic molecules by Treg cells with therapeutic potential. Analysis has been performed using flow cytometry (mean ± sem of n=12 Treg cell populations). **Figure 4** shows that Treg cells expressed at least 6 different cytotoxic molecules in the group of granulysin, granzyme A, granzyme B, granzyme K, granzyme H, granzyme M and perforin.

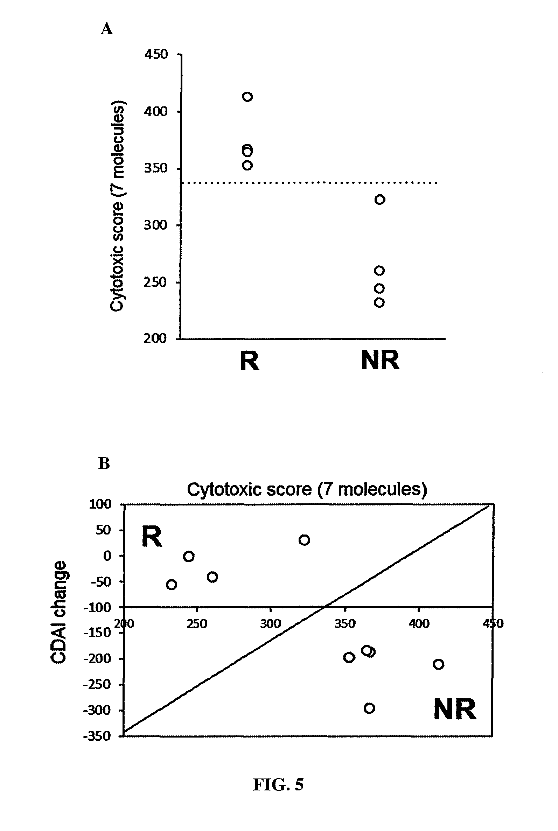

Moreover, **Figure 5** shows the "cytotoxic score" of Treg populations with therapeutic potential (groups of responder patients: R) and the "cytotoxic score" of Treg populations without therapeutic potential (groups of non-responder patients: NR). The cytotoxic score is represented by the sum of the % of cells in the Treg population expressing each of the 7 cytotoxic molecules from the group of granulysin, granzyme A, granzyme B, granzyme K, granzyme H, granzyme M and perforin. **Figure 5A** shows that a cytotoxic score above 340 identifies Treg cells with therapeutic potential whereas a cytotoxic score below 340 identifies Treg cells without therapeutic potential.

Moreover, **Figure 5B** shows that the therapeutic efficacy of Treg cells observed using the CDAI score in Crohn's Disease patients injected with Treg cells correlates with the cytotoxic score.

Moreover, Table 2 below shows the expression of 7 cytotoxic molecules from Treg cell populations with therapeutic efficacy (groups of responder patients: R, n=6) and from Treg cell populations without therapeutic efficacy (groups of non-responder patients: NR, n=4). This table demonstrates that in contrast to Treg cell populations without therapeutic efficacy all Treg cell populations with therapeutic efficacy expressed at least 3 cytotoxic molecules with at least 30% of cells expressing granzyme B and 80% of cells expressing granzyme H.

**Table 2**

| | % | % | % | % | % | % | % |
|---|---|---|---|---|---|---|---|
| **R** | **G** | **B** | **A** | **M** | **K** | **H** | **P** |
| A02 | 41.3 | 66.4 | 90.4 | 79.9 | 0.7 | 88.4 | 0.8 |
| A03 | 49.1 | 59.6 | 90.5 | 82.2 | 0 | 85.5 | 0.3 |
| E07 | 73.1 | 71.2 | 95.9 | 80.6 | 2.75 | 89.6 | 6.3 |
| A36 | 69.7 | 96.8 | 95.8 | 3 | 2.8 | 96.8 | 3.7 |
| D19 | 30.5 | 53.6 | 92.6 | 79.3 | 0.8 | 96.3 | 0.6 |
| | | | | | | | |

| | % | % | % | % | % | % | % |
|---|---|---|---|---|---|---|---|
| **NR** | **G** | **B** | **A** | **M** | **K** | **H** | **P** |
| E12 | 28.8 | 44.2 | 89.8 | 81.7 | 0.6 | 77.2 | 0.5 |
| C35 | 13.8 | 28.42 | 97.4 | 26.5 | 0 | 94 | 1.86 |
| D14 | 57.7 | 4 | 68.1 | 7.6 | 0.2 | 94.6 | 7.14 |
| D16 | 36.1 | 25.5 | 72.5 | 14.4 | 1.8 | 94.1 | 6.52 |

## Claims

1. A Treg cell population expressing at least 3 cytotoxic molecules selected from the group comprising granulysin, granzyme A, granzyme B, granzyme M, granzyme K, granzyme H and perforin.

2. The Treg cell population according to claim **1,** wherein said at least three cytotoxic molecules comprise granzyme B, preferably granzyme B and granzyme H.

3. The Treg cell population according to claim **1** or claim **2, characterized in that** the score resulting from the sum of:
- the percentages of cells of the Treg cell population expressing a first cytotoxic molecule, and
- the percentage of cells of the Treg cell population expressing a second cytotoxic molecule,
- the percentage of cells of the Treg cell population expressing a third cytotoxic molecule,
- optionally the percentage of cells of the Treg cell population expressing a fourth cytotoxic molecule,
- optionally the percentage of cells of the Treg cell population expressing a fifth cytotoxic molecule,
- optionally the percentage of cells of the Treg cell population expressing a sixth cytotoxic molecule, and
- optionally the percentage of cells of the Treg cell population expressing a seventh cytotoxic molecule,
is superior to a reference score measured in Treg cell populations that do not induce a therapeutic response when used in a Treg cell therapy in a patient.

4. The Treg cell population according to claim 1 or claim 2, wherein
- at least 30% of the cells of the Treg population express granzyme B,
- at least 80% of the cells of the Treg population express granzyme H, and
- at least 30% of the cells of the Treg population express at least one cytotoxic molecule selected from granulysin, granzyme A, granzyme K, granzyme M and perforin.

5. The Treg cell population according to anyone of claims 1 to 4, being a Tr1 cell population.

6. The Treg cell population according to anyone of claims 1 to 5, being an antigen-specific Treg cell population.

7. A method for identifying Treg cells according to claim 1, comprising determining the expression level of cytotoxic molecules, wherein said expression level of cytotoxic molecules comprises the expression level of at least 3 cytotoxic molecules selected from the group comprising granulysin, granzyme A, granzyme B, granzyme M, granzyme K, granzyme H and perforin.

8. The method according to claim 7, wherein said at least three cytotoxic molecules comprise granzyme B, preferably granzyme B and granzyme H.

9. The method according to anyone of claims 7 to 8, wherein said method comprises:
a. determining in the Treg cell population to be administered to the subject:
- the percentage of cells expressing a first cytotoxic molecule,
- the percentage of cells expressing a second cytotoxic molecule,
- the percentage of cells expressing a third cytotoxic molecule,
- optionally the percentage of cells expressing a fourth cytotoxic molecule,
- optionally the percentage of cells expressing a fifth cytotoxic molecule,
- optionally the percentage of cells expressing a sixth cytotoxic molecule, and
- optionally the percentage of cells expressing a seventh cytotoxic molecule,
b. summing the at least three percentages measured in step (a), thereby obtaining a score, and
c. comparing said score with a reference score.

10. The method according to anyone of claims 7 to 8, wherein a Treg cell population comprising:
- more than 30% of cells expressing granzyme B
- more than 80% of cells expressing granzyme H, and
- more than 30% of cells expressing at least one of granulysin, granzyme A, granzyme M, granzyme K and perforin,
is a Treg cell population with therapeutic potential.

11. The method according to anyone of claims 7 to 10, wherein the Treg cell population is a Tr1 cell population.

12. The method according to anyone of claims 7 to 11, wherein the Treg cell population is an antigen-specific Treg cell population.

13. A Treg cell population identified by the method of anyone of claims 7 to 12.

14. The Treg cell population according to anyone of claims 1 to 6 or 13, for use for treating an inflammatory condition, an autoimmune condition or an immune response associated with transplantation or with an allergic disease

15. A kit for implementing the method for identifying Treg according to anyone of claims 7 to 12, wherein said kit comprises means for measuring the expression of at least three, four, five, six or seven cytotoxic molecules, preferably wherein said means are antibodies specific for said at least three, four, five, six or seven cytotoxic molecules.
